# EUROPÄISCHE PATENTSCHRIFT

(11) **EP 1 175 240 B1**
(45) Veröffentlichungstag und Bekanntmachung des Hinweises auf die Patenterteilung: **01.09.2004**
(21) Anmeldenummer: 00931112.7
(22) Anmeldetag: 04.05.2000
(51) Int. Cl.: A61M 16/06, A61N 1/04

(54) **VORRICHTUNG ZUR ERFASSUNG ELEKTRISCHER POTENTIALE IM STIRNBEREICH EINES PATIENTEN**
DEVICE FOR DETECTING ELECTRICAL POTENTIALS IN THE FOREHEAD-AREA OF A PATIENT
DISPOSITIF DE DETECTION DE POTENTIELS ELECTRIQUES DANS LA ZONE FRONTALE D'UN PATIENT

(30) Priorität: 04.05.1999 DE 19920433; 05.08.1999 DE 19936505; 08.10.1999 DE 29917806 U; 26.11.1999 DE 19956841
(43) Veröffentlichungstag der Anmeldung: 30.01.2002
(73) Patentinhaber: MAP Medizin-Technologie GmbH, 82152 Martinsried (DE)
(72) Erfinder: GENGER, Harald, D-82319 Starnberg (DE); NEGELE, Claus, D-80801 München (DE)
(74) Vertreter: Rössig, Rolf
(86) Internationale Anmeldenummer: PCT/EP2000/003997
(87) Internationale Veröffentlichungsnummer: WO 2000/066209

(56) Entgegenhaltungen:
- WO-A-97/16216
- DE-A- 2 106 552
- US-A- 5 406 957
- US-A- 5 657 752
- US-A- 5 755 230

## Beschreibung

Die Erfindung betrifft eine Vorrichtung zur Erfassung elektrischer Potentiale im Stirnbereich eines Patienten.

Anhand derartiger elektrischer Potentiale ist es möglich Rückschlüsse auf die Gehirnaktivität eines Menschen zu ziehen. Insbesondere ist es möglich, bei einer schlafenden Person anhand der während des Schlafes ermittelten Gehimaktivität die einzelnen Schlafzustände zu bestimmen.

In der auf die Anmelderin zurückgehenden deutschen Patentanmeldung DE 1 99 20 433.0 ist ein CPAP-Gerätesystem beschrieben, bei welchem die Charakteristik der Atemgaszufuhr in Abhängigkeit vom Schlafzustand des Patienten verändert wird. Die erforderlichen Elektroden werden hierzu über einen Klebestreifen auf die Stirn des Patienten aufgeklebt.
Aus WO 97 16 216 A ist eine Vorrichtung zur Zufuhr eines Atemgases zu einem Patienten bekannt, gem. welcher die Zufuhr des Atemgases unter Überwachung physiologischer Parameter des Patienten erfolgt, wie beispielsweise der Körperposition, der Atmungstätigkeit sowie EEG-Signalen, insbesondere zur Ermittlung des Schlafzustandes eines Patenten.
Die korrekte Applikation dieser Elektroden erfordert besondere Sorgfalt und wird von den betroffenen Patienten häufig als unangenehm empfunden.

Der Erfindung liegt die Aufgabe zugrunde, die Zuverlässigkeit der Erfassung elektrischer Potentiale im Stirnbereich eines Patienten zu verbessern und die Applikation der erforderlichen Elektroden in einer für den Patienten angenehmen Weise zu ermöglichen.

Diese Aufgabe wird erfindungsgemäß gelöst durch eine Vorrichtung zur Erfassung elektrischer Potentiale an einem Patienten mit einer im Stirnbereich des Patienten applizierbaren Elektrodeneinrichtung, wobei die Elektrodeneinrichtung an einem Stirnauflageelement angeordnet ist das mit einer Atemmaskeneinrichtung derart zusammen wirkt, daß die Applikationsposition der Elektrodeneinrichtung im Zusammenhang mit der Applikationsposition der Atemmaskeneinrichtung festgelegt ist.

Dadurch wird es auf vorteilhafte Weise möglich mit hoher Wiederholgenauigkeit die Elektrodeneinrichtung lagerichtig zu applizieren.

Das Stirnauflageelement ist gemäß einer besonders bevorzugten Ausführungsform der Erfindung mit der Atemmaskeneinrichtung gekoppelt. In vorteilhafter Weise kann hierbei die Elektrodeneinrichtung unmittelbar mit der Atemmaskenanordnung appliziert bzw. abgenommen werden. Auf die bisher erforderlichen Klebstoffstreifen kann auf vorteilhafte Weise verzichtet werden.

Eine besonders hohe Anpassungsfähigkeit des Stirnauflageelementes an die individuelle Gestalt der Stirnpartie des Patienten sowie ein hoher Tragekomfort ist gemäß einer besonders bevorzugten Ausführungsform der Erfindung dadurch gegeben, daß das Stirnauflageelement aus einem elastomeren Material gebildet ist.

Eine besonders gewichtsparende und unter hygienischen Gesichtspunkten vorteilhafte Ausführungsform der Erfindung ist dadurch gegeben, daß das Stirnauflageelement einstückig mit einem Maskenbasiskörper der Atemmaskeneinrichtung ausgebildet ist.

Die räumliche Gestalt der aus Stirnauflageelement und Maskenkörper gebildeten Einheit kann gemäß einem besonderen Aspekt der vorliegenden Erfindung an die individuelle Gesichtskontur des Patienten angepaßt werden indem ein Versteifungselement vorgesehen ist, das das Stirnauflageelement und die Atemmaskeneinrichtung versteifend miteinander koppelt.

Die Elektrodeneinrichtung umfaßt vorzugsweise wenigstens zwei Elektrodenelemente wobei der Potentiatunterschied zwischen diesen beiden Elektrodenelementen erfaßt wird. Eine erheblich genauere Bestimmung der hirnelektrischen Aktivität des Patienten wird möglich, indem die Elektrodeneinrichtung wenigstens drei Elektrodenelemente aufweist. Diese Elektrodenelemente werden beispielsweise in den frontalen Meßstellen fP1 und fP2 sowie einer dazwischenliegenden Stelle plaziert.

Vorzugsweise befinden sich die Meßstellen im Stirnbereich des Patienten ca. 30mm oberhalb der Augenbrauen.

Eine besonders hohe Meßgenauigkeit wird in vorteilhafter Weise dadurch erreicht, daß die Elektrodenelemente in eine zur Applikationsfläche im wesentlichen senkrechte Richtung nachgiebig gelagert sind. Dadurch wird vermieden, daß beispielsweise in Abhängigkeit von einer Stirnbandspannung unterschiedliche Elektrodenanpreßkräfte hervorgerufen werden. Beispielsweise sind die Elektrodenelemente hierzu in einer Topfstruktur aufgenommen. Die Nachgiebigkeit kann durch elastomere Elemente insbesondere Rollbalgmembranen erreicht werden. Alternativ hierzu ist es auch möglich, die Elektrodenelemente in einer Versenkung aufzunehmen, wobei die Tiefe der Versenkung im wesentlichen der Dicke der Elektrodenelemente entspricht.

Die Elektrodenelemente sind gemäß einer besonders bevorzugten Ausführungsform der Erfindung mit einer Signalverarbeitungseinrichtung gekoppelt die in unmittelbarer Nähe der Elektrodenelemente angeordnet ist. Die Signalverarbeitungseinrichtung ist hierzu in besonders vorteilhafter Weise in das Stirnauflageelement integriert und weist eine eigene Spannungsversorgungseinrichtung beispielsweise in Form einer Knopfzelle auf. Der Ausgang der Signalverarbeitungseinrichtung ist vorzugsweise potentialfrei und kommuniziert unmittelbar mit einer Datenübertragungseinrichtung, zur schnurlosen insbesondere Funk-Übertragung der verarbeiteten Signale an eine Datenverarbeitungseinrichtung. Es ist möglich, die über die Elektrodenelemente erfaßten Potentiale noch im Bereich des Stirnauflageelementes durch eine entsprechenden Datenverarbeitungseinrichtung zu verarbeiten so daß ein komprimierter oder aussagefähigerer Datensatz erzeugt wird der mit geringerem Übertragungsaufwand weitergeleitet werden kann. Andererseits ist es auch möglich im wesentlichen nur die Rohdaten d.h. die Meßergebnisse an eine separate Empfangseinrichtung weiterzuleiten.

Diese Empfangseinrichtung kann Teil eines Patientenüberwachungssystems eines Schlaflabors sein. Es ist auch möglich die Empfangseinrichtung unmittelbar in ein CPAP Gerät zu integrieren. Insbesondere bei dieser Ausführungsform ist es auch möglich anstelle einer telemetrischen Signalübertragung eine Datenleitung vorzusehen die in besonders vorteilhafter Weise in einen Atemgasschlauch integriert ist.

Insbesondere im Hinblick auf den Anwendungsbereich der Schlaftherapie zur Behandlung schlafbezogener Atmungsstörungen wird die eingangs angegebene Aufgabe auch gelöst durch eine Atemmaskenanordnung zur Zufuhr eines Atemgases zu einem Patienten unter Überdruck, mit einem den Nasenbereich des Patienten übergreifenden Maskenkörper, einer Abdichtungseinrichtung zur Abdichtung eines Maskeninnenbereiches gegenüber der Umgebung, und einem Stirnauflageelement zum Abstützen des Maskenkörpers im Stirnbereich des Patienten, dadurch gekennzeichnet, daß im Bereich des Stirnauflageelementes eine Elektrodeninrichtung vorgesehen ist, zur Erfassung elektrischer, insbesondere hirnelektrischer Potentiale.

Das Stirnauflageelement hierbei ebenfalls wie bereits näher erläutert vorzugsweise aus einem elastomeren Material gebildet und entweder integral mit dem Maskenkörper ausgebildet oder über eine entsprechende Fügestruktur definiert mit diesem gekoppelt.

Insbesondere bei der integralen Ausgestaltung von Stirnauflageelement und Maskenörper ist dieser ebenfalls aus einem elastomeren Material gebildet. Insbesondere bei dieser Ausführungsform sind das Stirnauflageelement und der Maskenkörper mit einer Aussteifung versehen die sich bis in das Stirnauflageelement hinein erstreckt, wobei die Aussteifung vorzugsweise an die individuelle Gesichtskontur des Patienten angepaßt ist.

In Kombination mit den vorangehend beschriebenen Maßnahmen, oder auch alternativ hierzu wird die eingangs angegebene Aufgabe auch gelöst durch eine Vorrichtung zur Erfassung elektrischer Potentiale im Stirnbereich eines Patienten insbesondere zur Schlafstadienbestimmung, mit einer Elektrodeneinrichtung, einer Meßschaltungsanrdnung zur Erzeugung von Meßdaten nach Maßgabe der durch die Elektrodeneinichtung detektierten elektrischen Potentiale, wobei die Meßschaltungsanordnung in ein Stirnauflageelement integriert ist, und eine Signalübertragungseinrichtung vorgesehen ist, zur schnurlosen Übertragung der durch die Meßschaltungsanordnung erzeugten Meßdaten.

Die Meßschaltungsanordnung weist vorzugsweise eine Einrichtung zur Datenkomprimierung auf, zur Weiterleitung eines komprimierten Datensatzes an die Signalübertragungsenrichtung.

Eine gemäß einem weiteren Aspekt der vorliegenden Erfindung vorteilhafte Lösung der eingangs genannten Aufgabe ist erfindungsgemäß gegeben durch eine Vorichtung zur Erfassung elektrischer Potentiale im Stirnbereich eines Patienten insbesondere zur Schlafstadienbestimmung, mit einer Elektrodeneinrichtung, einer Meßschaltungsanordnung zur Erzeugung von Meßdaten nach Maßgabe der durch die Elektrodeneinrichtung detektierten elektrischen Potentiale, wobei die Meßschaltungsanordnung in ein Stirnauflageelement integriert ist, und eine Meßdatenaufzeichnungseinrichtung vorgesehen ist, zur Aufzeichnung der durch die Meßschaltungsanordnung erzeugten Meßdaten.

Diese Meßdatenaufzeichnungseinrichtung umfaßt in besonders vorteilhafter Weise ein in etwa briefmarkengroßes Speicherkartenelement das lösbar mit dem Stimauflageeement gekoppelt ist und das zur weiteren Verarbeitung der aufgezeichneten Informationen beispielsweise aus dem Stirnauflageelement entfernt werden kann. Die Meßdaten können ggf. durch eine im Bereich des Stirnauflageelementes vorgesehene Komprimierungseinrichtung komprimiert werden.

Weitere vorteilhafte Ausgestaltungen der Erfindung sind in den Unteransprüchen dargelegt.

Weitere Einzelheiten ergeben sich aus der nachfolgenden Beschreibung mehrerer bevorzugter Ausführungsformen der Erfindung sowie hierbei in vorteilhafter Weise verwirklichten Detail-Lösungen unter Bezugnahme auf die beigefügte Zeichnung.

### Es zeigen:

- **Fig. 1**: eine perspektivische Ansicht einer Atemmaske mit einem Stirnauflageelement, das drei Elektroden zur Erfassung elektrischer Potentiale im Stirnbereich des Patienten aufweist;
- **Fig. 2**: eine vereinfachte Schnittansicht zur Erläuterung einer elastisch nachgiebigen Lagerung eines Elektrodenelementes;
- **Fig. 3a**: eine perspektivische Ansicht eines elastomeren Bandelementes, das drei Elektroden zur Erfassung der hirnelektrischen Aktivität eines Patienten aufweist, wobei das Bandelement mit einem Stirnauflageelement koppelbar ist;
- **Fig. 3b**: eine vereinfachte Schnittansicht durch ein Stirnauflageelement, in welches das in Fig. 3a dargestellte Bandelement eingesetzt ist;
- **Fig. 3c**: eine vereinfachte Schnittansicht durch ein Elektrodenelement, wie es insbesondere bei dem Stirnauflageelement gem. Fig. 3a zur Anwendung kommt;
- **Fig. 4**: eine perspektivische Ansicht einer weiteren Ausführungsform eines Stirnauflageelementes mit integrierten Elektrodenelementen zur Erfassung der hirnelektrischen Aktivität eines Patienten, wobei das Stirnauflageelement mit einem Kanalabschnitt einer Atemmaske koppelbar ist;
- **Fig. 5**: eine vereinfachte Darstellung zur Erläuterung eines Stirnbandelementes mit integrierter Signalverarbeitungseinrichtung;
- **Fig. 6a**: eine perspektivische Ansicht einer zum Einsatz in ein Stirnauflagelement vorgesehenen Signalverarbeitungseinrichtung entweder wie im Falle a zur telemetrischen (beispielsweise Funk-) Datenübertragung oder Fall b zur Abspeicherung eines vorzugsweise komprimierten Datensatzes auf einem auswechselbaren Datenträger vorzugsweise in Chip-Kartenform;
- **Fig. 6b**: eine vereinfachte Schnittansicht durch einen in einem Stirnauflagelement ausgebildeten Aufnahmeabschnitt zur Aufnahme der Signalverarbeitungseinrichtung gem. Fig. 6a;
- **Fig. 7**: eine vereinfachte Darstellung einer erfindungsgemäßen Meßanordnung zur Erfassung der hirnelektrischen Aktivität eines Patienten hiermit insgesamt fünf über eine Atemmaske vorpositionierten Elektrodenelementen sowie einer telemetrischen Datenübertragungseinrichtung.

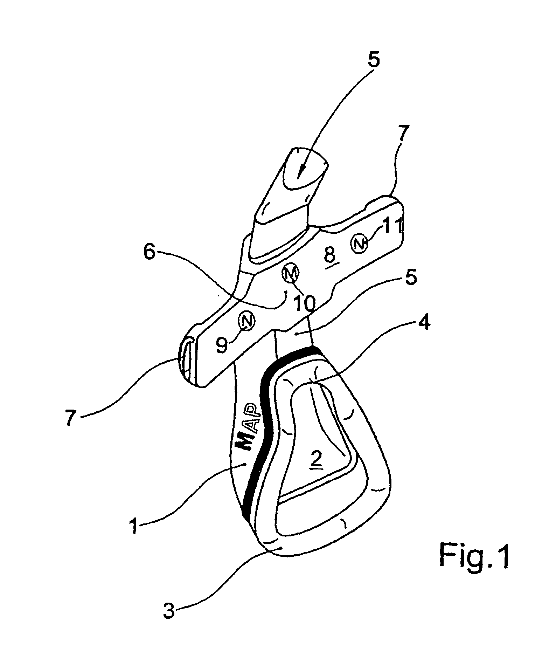

Die in Fig. 1 dargestellte Atemmaske umfaßt einen Maskenkörper 1, der einen Maskeninnenraum 2 begrenzt. An einer dem Umgebungsbereich der Nase eines Patienten zugewandten Kantenabschnitt ist eine Dichtungseinrichtung 3 vorgesehen, die hier wenigstens eine elastische Dichtlippe aufweist, die mit der Gesichtsfläche des Patienten in innige Anlage gelangt und dabei den Maskeninnenraum 2 gegenüber der Umgebung abdichtet. Die Dichtungseinrichtung 3 weist hier einen im Nasenrückenbereich vergleichsweise tief eingezogenen Abschnitt 4 auf, wodurch eine mit hoher Wiederholgenauigkeit gleichbleibende Plazierung des Maskenkörpers 1 gegenüber der Nase des Patienten erreicht wird.

Der Maskenkörper 1 weist bei der hier dargestellten Ausführungsform einen Atemgaskanal 5 auf, der sich durch ein Stirnauflageelement 6 hindurch erstreckt. Bei der hier dargestellten Ausführungsform sind der Maskenkörper 1 und das Stirnauflageelement 6 integral aus einem elastomeren Material, insbesondere einem volltransparenten Silikonkautschukmaterial gebildet.

Das Stirnauflageelement 6 ist bei der dargestellten Ausführungsform mit Anschlußorganen 7 versehen, über welche das Stirnauflageelement 6 mit einem vorzugsweise gepolsterten Stirnband koppelbar ist. Die der Stirn des Patienten zugewandte Auflagefläche 8 des Stirnauflageelementes 6 ist bei der dargestellten Ausführungsform schwach konkav ausgebildet und zudem durch ein hier nicht näher dargestelltes Versteifungselement an die individuelle Kontur des Stirnbereiches des Patienten in ihrer Krümmung angepaßt. In der Auflagefläche 8 sind bei der hier dargestellten Ausführungsform insgesamt drei Elektrodenelemente 9, 10, 11 angeordnet, über welche elektrische Potentiale im Stirnbereich des Patienten erfaßt werden können.

Die Position der Elektrodenelemente 9, 10, 11 relativ zum Patienten kann mit hoher Wiederholgenauigkeit beibehalten werden, da die durch den Maskenkörper 1 und das Stirnauflageelement 6 gebildete Einheit insbesondere durch den Nasenbereich des Patienten präzise festgelegt ist.

Bei der hier dargestellten Ausführungsform weist der Atemgaskanal 5 einen unrunden, insbesondere polygonalen Querschnitt auf und ist zudem entsprechend der Wölbung der Stirn des Patienten zum Patienten hin abgekrümmt ausgebildet. Durch die hier verwendete Querschnittsform wird eine weitgehend wirbelfreie Einströmung des Atemgases in den Maskeninnenraum 2 erreicht. Diese Maßnahme stellt eine eigenständige neuartige Lösung zur Verbesserung der Atemgasströmung dar die auch unabhängig von den ansonsten beschriebenen Einzelheiten eigenständig verwirklicht werden kann.

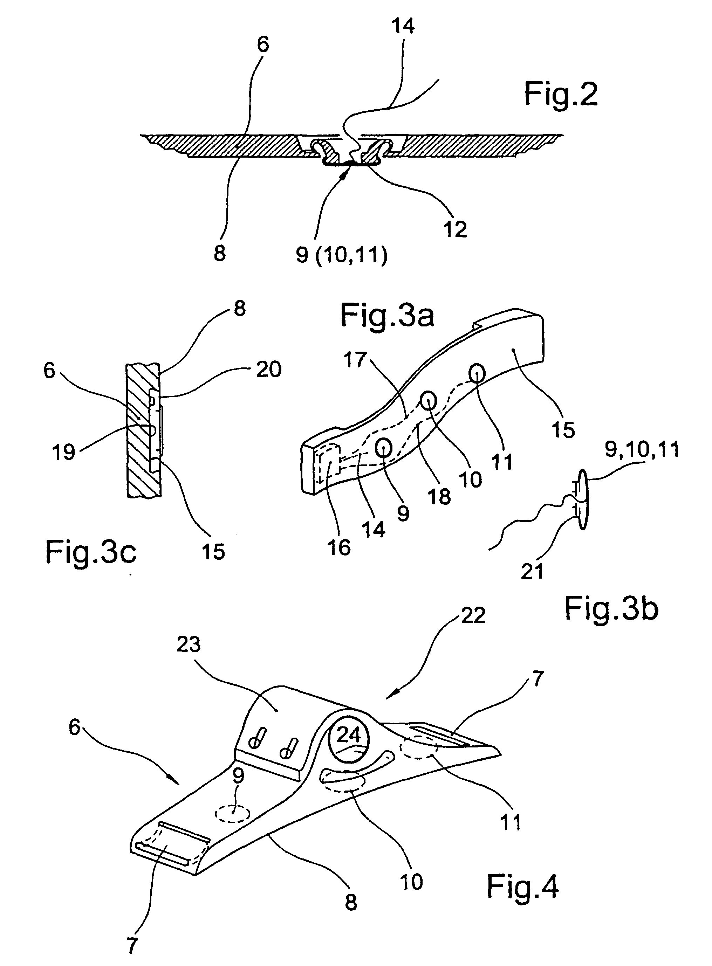

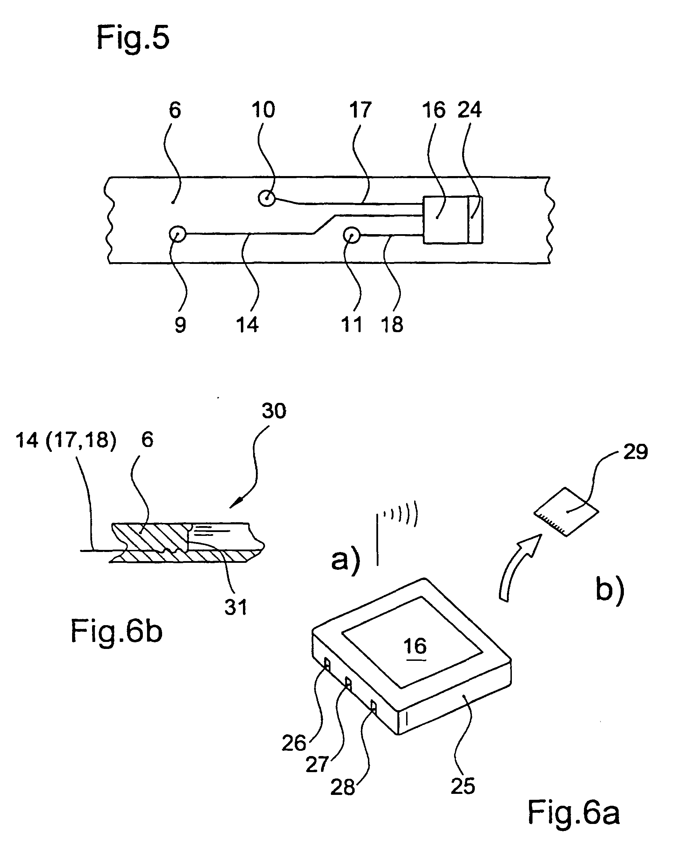

In Fig. 2 ist anhand einer vereinfachten Skizze eine bevorzugte Ausführungsform der Lagerung der Elektrodenelemente 9, 10, 11, wie sie bei der Atemmaske gem. Fig. 1 Anwendung finden, dargestellt. Wie erkennbar, sind die Elektrodenelemente 9, 10, 11 durch ein dünnes Metallplättchen 12 gebildet, das im wesentlichen senkrecht zur Auflagefläche 8 nachgiebig gelagert ist. Die nachgiebige Lagerung wird hierdurch eine integral mit dem Stirnauflageelement 6 ausgebildete Membranstruktur erreicht. Im rückwärtigen Bereich des Metallplättchens 12 ist eine hochflexible metallische Leitungseinrichtung 14 vorgesehen, die mit einer nachfolgend in Verbindung mit den Fig. 3a und 5 noch näher beschriebenen Ausführungsform erläutert werden wird. Durch die in Fig. 2 gezeigte elastisch nachgiebige Lagerung der Elektrodenelemente 9, 10, 11 wird erreicht, daß die Elektrodenelemente mit einer weitgehend gleichbleibenden Andruckkraft gegen die Hautoberfläche des Patienten gedrängt werden, wodurch infolge unterschiedlicher Kräfte in der Stirnbandanordnung ggf. verfälschte Meßergebnisse vermieden werden.

In Fig. 3b ist eine besondere Ausführungsform der erfindungsgemäßen Elektrodenanordnung gezeigt, die hier ein aus einem Elastomermaterial gebildetes Band 15 aufweist, in das die Elektrodenelemente 9, 10, 11 eingesetzt sind. Das Band 15 weist eine Außenkontur auf, die eine positionsgenaue Koppelung mit einem Stirnauflageelement ermöglicht. In einem Endabschnitt des Bandes 15 ist eine Signalverarbeitungseinrichtung 16 eingesetzt, die über dünne Verbindungsleitungen 14, 17, 18 mit den jeweiligen Elektrodenelementen 9, 10, 11 in Verbindung steht.

In dieser Signalverarbeitungseinrichtung werden die Potentialunterschiede zwischen den jeweiligen Elektrodenelementen erfaßt und ausgewertet. Die so gewonnenen Meßdaten werden in entsprechend kodierter Form entweder gespeichert oder telemetrisch an eine Empfangseinrichtung zur weiteren Datenverarbeitung geleitet.

In Fig. 3c ist eine Schnittansicht durch einen Ausschnitt eines Stirnauflageelementes 6 dargestellt, in welches das in Fig. 3a angesprochene Band 15 eingesetzt ist. Hierzu ist in dem Stirnauflageelement 6 eine Ausnehmung 19 ausgebildet. Die gegenüber der Auflagefläche 8 zurückfallende Tiefe der Ausnehmung 19 ist derart bemessen, daß die durch das Band 15 gebildete Auflagefläche 20 im wesentlichen bündig mit der Auflagefläche 8 abschließt.

An dem Band 15 sind - wie in Fig. 3a bereits angedeutet - die Elektrodenelemente 9, 10 und 11 angebracht.

Die Befestigung der Elektrodenelemente kann beispielsweise - wie in Fig. 3b angedeutet - erreicht werden, indem die Elektrodenelemente auf ihrer Rückseite nadelartige Vorsprünge 21 aufweisen, über welche die Elektrodenelemente individuell an dem Band 15 bzw. unmittelbar an dem Stirnauflageelement 6 plaziert werden können.

Alternativ dazu ist es auch möglich, in dem Stirnauflageelement 6 oder in dem Band 15 flache Ausnehmungen auszubilden, die von einem Umfangsrand begrenzt sind, in welchen die Elektrodenelemente 9, 10, 11 unter einem leichten Klemmsitz einklippsbar sind.

In Fig. 4 ist eine perspektivische Ansicht eines Stirnauflageelementes 6 dargestellt, das mit einem Atemgaskanalabschnitt einer Atemmaske koppelbar ist. Hierzu weist das Stirnauflageelement 6 einen Maskenbefestigungsabschnitt 22 auf. Dieser Maskenbefestigungsabschnitt umfaßt bei der hier dargestellten Ausführungsform ein flexibles Band 23, durch welches ein Durchgangskanal 24 zur Aufnahme des Atemgaskanales 5 begrenzt ist.

Das Stirnauflageelement 6 weist ferner Anschlußorgane 7 auf, die - wie bereits in Verbindung mit Fig. 1 angesprochen - zum weiteren Anschluß eines Stirnbandes dienen. Im Bereich der Auflagefläche 8 des Stirnauflageelementes 6 sind wiederum die Elektrodenelemente 9, 10 und 11 angebracht, die infolge der Koppelung der Atemmaske mit dem Stirnauflageelement 6 wiederholbar positionsgenau im Stirnbereich des entsprechenden Patienten appliziert werden können.

In Fig. 5 ist vereinfacht angedeutet, wie die einzelnen Elektrodenelemente 9, 10, 11 über die Verbindungsleitungen 14, 17, 18 mit der Signalverarbeitungseinrichtung 16 gekoppelt sind. Die Signalverarbeitungseinrichtung 16 umfaßt hier eine unmittelbar angeschlossene Datenübertragungseinrichtung 24, über welche die gewonnenen Meßdaten zur weiteren Verarbeitung an ein entsprechendes System schnurlos weitergeleitet werden können. Bei der hier dargestellten Ausführungsform sind sämtliche der genannten Komponenten in abdichtender Weise in dem aus einem Elastomermaterial gebildeten Basiskörper des Stirnauflageelementes 6 eingebettet.

In Fig. 6a ist eine besonders bevorzugte Ausführungsform der Signalverarbeitungseinrichtung 16 dargestellt, die hier in einem Außengehäuse 25 aufgenommen ist und mehrere Kontaktelemente 26, 27, 28 aufweist, die mit den in Verbindung mit Fig. 5 angesprochenen Verbindungsleitungen 14, 17 und 18 elektrisch verbindbar sind. Die hier dargestellte Verarbeitungseinrichtung 16 umfaßt eine eigene, durch eine Knopfzelle (nicht dargestellt) gebildete Spannungsversorgungseinrichtung und erzeugt in Abhängigkeit von den zwischen den Kontaktelementen 26, 27 und 28 erfaßten Potentialunterschieden binär kodierte Daten. Diese Daten können entweder, wie für den Fall a angedeutet, in Form von elektromagnetischen Wellen, insbesondere Funk, an ein externes Datenverarbeitungssystem übertragen werden. Alternativ dazu oder auch in Kombination hiermit ist es möglich - wie für den Fall angedeutet dargestellt - Die gewonnenen Meßdaten auf einem Datenträger 29 abzuspeichern. Um eine unmittelbare zeitliche Zuordnung dieser Meßdaten zu erhalten, kann die Signalverarbeitungsvorrichtung 16 gem. einer besonders bevorzugten Ausführungsform der Erfindung mit einer Uhr versehen sein, so daß die erzeugten Meßdaten auch entsprechend zeitlicher Zuordnung abgespeichert werden.

Die als vollständig abgeschlossener Block ausgebildete Signalverarbeitungseinrichtung 16 kann - wie in Fig. 6b angedeutet - in eine Ausnehmung 30 eingeklippst werden, die unmittelbar in dem Stirnauflageelement 6 oder wie beispielsweise im Fall der Fig. 3a angedeutet, in ein entsprechendes Band 15 eingeklippst ist. Die an der Signalverarbeitungseinrichtung 16 ausgebildeten Kontaktelemente 26, 27 und 28 gelangen hierbei aufgrund der Eigenelastizität des die Ausnehmung 30 umgebenden Materiales in einen hinreichend hohen Preßkontakt mit den weiteren in dem Stirnauflageelement 6 vorgesehenen Gegenkontakten 31. Diese Gegenkontakte 31 sind mit den bereits genannten Signalleitungen 14, 17, 18 verbunden.

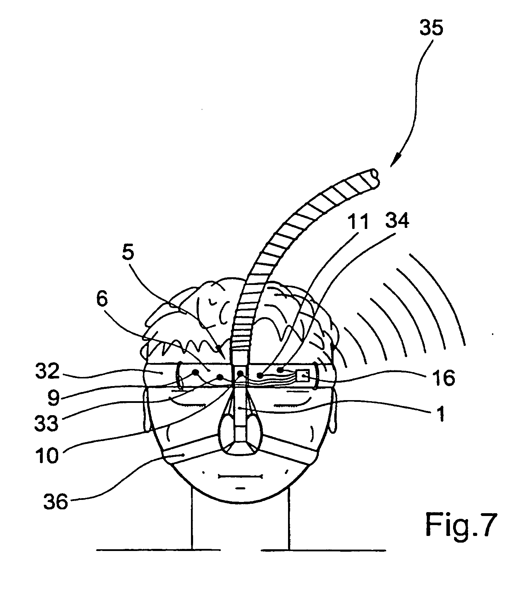

In Fig. 7 ist vereinfacht dargestellt, wie eine erfindungsgemäße Atemmaske mit integrierten Stirnelektroden im Gesichtsbereich eines Patienten appliziert ist. Hierbei wird deutlich, daß durch die erfindungsgemäße Koppelung der Elektrodenelemente mit einem zur Abstützung einer Atemmaske vorgesehenen Stirnauflageelement eine extrem präzise Positionierung der Elektrodenelemente erreicht wird. Hierdurch wird es möglich, mehrere Elektrodenelemente, insbesondere, wie hier dargestellt, fünf Elektrodenelemente im Stirnbereich des Patienten zu applizieren, wobei bei jeder Neuapplikation der Elektrodenelemente im wesentlichen wieder die gleiche Meßposition wie bei den vorangegangenen Messungen erreicht wird. Hierdurch wird eine erheblich verbesserte Vergleichbarkeit der im Rahmen von unterschiedlichen Meßzyklen gewonnenen Daten erreicht.

Die in Fig. 7 dargestellte Meßanordnung umfaßt den Maskenkörper 1 und das damit über den Atemgaskanalabschnitt 5 gekoppelte Stirnauflageelement 6, das hier lediglich andeutungsweise dargestellt ist. In dem Stirnauflageelement 6 sind hier insgesamt fünf Elektroden 9, 10, 11 sowie 33 und 34 angeordnet, über welche elektrische Potentialunterschiede im Stirnbereich des Patienten erfaßt werden können.

Die so erfaßten Potentialunterschiede werden von der in das Stirnauflageelement 6 integrierten Signalverarbeitungseinrichtung 16 ausgewertet und über Funksignale an eine externe Empfangseinrichtung zur weiteren Verarbeitung insbesondere zur Steuerung eines CPAP-Gerätes weitergeleitet.

Die Zufuhr des Atemgases zu dem durch den Maskenkörper 1 begrenzten Maskeninnenraum erfolgt hier über einen Atemgasschlauch 35, der mit einer spiralartig ausgebildeten Verstärkungseinlage versehen ist.

Insbesondere bei einem derartigen Atemschlauch ist es möglich, anstelle der Übertragung der durch die Signalverarbeitungseinrichtung 16 erzeugten Signale über Funk eine Übertragung der Signale durch eine Leitungseinrichtung vorzunehmen, die in den Atemgasschlauch 35 insbesondere in dessen Verstärkungsspirale integriert ist.

Der dem Mund des Patienten benachbarte Bereich des Maskenkörpers wird durch eine untere Gurtbandanordnung 36 mit einer vorbestimmten Andruckkraft gegen das Gesicht des Patienten gedrängt. Das Stirnauflageelement 6 wird durch eine um den Hinterkopfbereich des Patienten umlaufende obere Gurtbandanordnung 37 mit einer einstellbaren Anpreßkraft gegen die Stirn des Patienten gedrängt. Sowohl die untere Gurtbandanordnung 36 als auch die obere Gurtbandanordnung 37 sind bei der hier dargestellten Ausführungsform durch ein aus einem vergleichsweise zugsteifen, gepolsterten Bandmaterial gebildet. Durch die hohe Zugsteifigkeit dieses Bandmateriales wird erreicht, daß auch bei vergleichsweise geringen Anpreßkräften der Atemmaske auf das Gesicht des Patienten diese Atemmaske infolge des im Maskeninnenbereichs herrschenden Überdruckes nicht vom Gesicht des Patienten abhebt. Im Bereich der Koppelungsstelle zwischen dem Atemgasschlauch 35 und dem Atemgaskanalabschnitt 5 der Atemmaske kann ggf. eine Kugelgelenkeinrichtung vorgesehen sein, durch welche die Einleitung etwaiger Drehmomente in die Atemmaske noch weiter unterdrückt wird.

## Patentansprüche

1. Vorrichtung zur Erfassung elektrischer Potentiale an einem Patienten mit einer an Patienten applizierbaren Elektrodeneinrichtung, (9,10,11) **dadurch gekennzeichnet daß**, die Elektrodeneinrichtung (9,10,11) an einem Stirnauflageelement (6) angeordnet ist das mit einer Atemmaskeneinrichtung (1,3) derart zusammen wirkt, daß die Applikationsposition der Elektrodeneinrichtung (9,10,11) im Stimbereich im Zusammenhang mit der Applikationsposition der Atemmaskeneinrichtung (1,3) festgelegt ist.

2. Vorrichtung nach Anspruch 1, **dadurch gekennzeichnet, daß** das Stirnauflageelement (6) mit der Atemmaskeneinrichtung (1,3) gekoppelt ist.

3. Vorrichtung nach Anspruch 1 oder 2, **dadurch gekennzeichnet, daß** das Stirnauflageelement (6) aus einem elastomeren Material gebildet ist.

4. Vorrichtung nach einem der Ansprüche 1 - 3, **dadurch gekennzeichnet, daß** das Stirnauflageelement (6) einstückig mit einem Maskenbasiskörper (1) der Atemmaskeneinrichtung (1,3) ausgebildet ist.

5. Vorrichtung nach einem der Ansprüche 1 - 4, **dadurch gekennzeichnet, daß** ein Versteifungselement vorgesehen ist, das das Stirnauflageelement (6) und die Atemmaskeneinrichtung (1,3) versteifend miteinander koppelt.

6. Vorrichtung nach einem der Ansprüche 1 - 5, **dadurch gekennzeichnet, daß** die Elektrodeneinrichtung (9,10,11) wenigstens zwei Elektrodenelemente (9,10) aufweist.

7. Vorrichtung nach einem der Ansprüche 1 - 6, **dadurch gekennzeichnet, daß** die Elektrodeneinrichtung (9,10,11) drei Elektrodenelemente (9,10,11) aufweist.

8. Vorrichtung nach einem der Ansprüche 1 - 7, **dadurch gekennzeichnet, daß** die Elektrodenelemente in eine (9,10,11) zur Applikationsfläche im wesentlichen senkrechte Richtung nachgiebig gelagert sind.

9. Vorrichtung nach einem der Ansprüche 1 - 8, **dadurch gekennzeichnet, daß** die Elektrodenelemente (9,10,11) mit einer Signalverarbeitungseinrichtung (16) gekoppelt sind.

10. Vorrichtung nach einem der Ansprüche 1 - 9, **dadurch gekennzeichnet, daß** die Signalverarbeitungseinrichtung (16) in das Stirnauflageelement (6) integriert ist.

11. Vorrichtung nach einem der Ansprüche 1 - 10, **dadurch gekennzeichnet, daß** die Signalverarbeitungseinrichtung (16) mit einer Datenübertragungseinrichtung versehen ist, zur schnurlosen Übertragung der verarbeiteten Signale an eine Datenverarbeitungseinrichtung.

12. Vorrichtung nach einem der Ansprüche 1 - 11, **dadurch gekennzeichnet, daß** der Maskenkörper (1) und das Stirnauflageelement (6) durch Aussteifung mit einer sich bis in das Stirnauflageelement (6) hinein erstreckenden Versteifungseinrichtung an die individuelle Gesichtskontur des Patienten angepaßt sind.

13. Vorrichtung noch wenigstens einem der Ansprüche 1 bis 12, zur Erfassung elektrischer Potentiale im Stirnbereich eines Patienten insbesondere zur Schlafstadienbestimmung, mit:
einer Meßschaltungsanordnung zur Erzeugung von Meßdaten nach Maßgabe der durch die Elektrodeneinrichtung (9,10,11) detektierten elektrischen Potentiale, wobei die Meßschaltungsanordnung in ein Stirnauflageelement (6) integriert ist, und eine Signalübertragungseinrichtung (16) vorgesehen ist, zur schnurlosen Übertragung der durch die Meßschaltungsanordnung erzeugten Meßdaten.

14. Vorrichtung nach Ansprüch 13, **dadurch gekennzeichnet, daß** die Meßschaltungsanordnung eine Einrichtung zur Datenkomprimierung aufweist, zur Weiterleitung eines komprimierten Datensatzes an die Signalübertragungseinrichtung (16).

15. Vorrichtung nach wenigstens einem der Ansprüche 1 bis 12 zur Erfassung elektrischer Potentiale im Stimbereich eines Patienten insbesondere zur Schlafstadienbestimmung, mit:
einer Meßschaltungsanordnung zur Erzeugung von Meßdaten nach Maßgabe der durch die Elektrodeneinrichtung (9, 10, 11) detektierten elektrischen Potentiale, wobei die Meßschaltungsanordnung in ein Stirn-auflageelement (6) integriert ist, und eine Meßdatenaufzeichnungseinrichtung (29) vorgesehen ist, zur Aufzeichnung der durch die Meßschaltungsanordnung erzeugten Meßdaten.

16. Vorrichtung nach Ansprüche 15, **dadurch gekennzeichnet, daß** die Meßdatenaufzeichnungseinrichtung (29) durch ein in etwa briefmarkengroßes Speicherkartenelement gebildet ist, das lösbar angebracht ist.

## Claims

1. A device for detecting electrical potentials on a patient, with an electrode device (9, 10, 11) which can be applied to the patient, **characterised in that** the electrode device (9, 10, 11) is arranged on a forehead support element (6) which co-operates with a breathing mask device (1, 3) in such a way that the application position of the electrode device (9, 10, 11) in the forehead region is established in conjunction with the application position of the breathing mask device (1, 3).

2. A device according to claim 1 **characterised in that** the forehead support element (6) is coupled to the breathing mask device (1, 3).

3. A device according to claim 1 or claim 2 **characterised in that** the forehead support element (6) is formed from an elastomer material.

4. A device according to one of claims 1 to 3 **characterised in that** the forehead support element (6) is formed in one piece with a mask base member (1) of the breathing mask device (1, 3).

5. A device according to one of claims 1 to 4 **characterised in that** there is provided a stiffening element which stiffeningly couples together the forehead support element (6) and the breathing mask device (1, 3).

6. A device according to one of claims 1 to 5 **characterised in that** the electrode device (9, 10, 11) has at least two electrode elements (9, 10).

7. A device according to one of claims 1 to 6 **characterised in that** the electrode device (9, 10, 11) has three electrode elements (9, 10, 11).

8. A device according to one of claims 1 to 7 **characterised in that** the electrode elements (9, 10, 11) are mounted yieldingly in a direction substantially perpendicular to the application surface.

9. A device according to one of claims 1 to 8 **characterised in that** the electrode elements (9, 10, 11) are coupled to a signal processing device (16).

10. A device according to one of claims 1 to 9 **characterised in that** the signal processing device (16) is integrated into the forehead support element (6).

11. A device according to one of claims 1 to 10 **characterised in that** the signal processing device (16) is provided with a data transmission device for the cord-less transmission of the processed signals to a data processing device.

12. A device according to one of claims 1 to 11 **characterised in that** the mask member (1) and the forehead support element (6) are adapted to the individual contour of the face of the patient by virtue of stiffening with a stiffening device which extends into the forehead support element (6).

13. A device according to at least one of claims 1 to 12 for detecting electrical potentials in the forehead region of a patient, in particular for determining sleep stages, comprising:
a measuring circuit arrangement for producing measurement data in accordance with the electrical potentials detected by the electrode device (9, 10, 11), wherein the measuring circuit arrangement is integrated into a forehead support element (6), and there is provided a signal transmission device (16) for cord-less transmission of the measurement data produced by the measuring circuit arrangement.

14. A device according to claim 13 **characterised in that** the measuring circuit arrangement has a data compression device for forwarding a compressed data set to the signal transmission device (16).

15. A device according to at least one of claims 1 to 12 for detecting electrical potentials in the forehead region of a patient, in particular for determining sleep stages, comprising:
a measuring circuit arrangement for producing measurement data in accordance with the electrical potentials detected by the electrode device (9, 10, 11), wherein the measuring circuit arrangement is integrated into a forehead support element (6), and there is provided a measurement data recording device (29) for recording the measurement data produced by the measuring circuit arrangement.

16. A device according to claim 15 **characterised in that** the measurement data recording device (29) is formed by an approximately postage stamp-size memory card element which is releasably fitted.

## Revendications

1. Dispositif de détection de potentiels électriques sur un patient comportant un dispositif d'électrodes (9, 10, 11) applicable au patient, **caractérisé en ce que** le dispositif d'électrodes (9, 10, 11 ) est disposé sur un élément d'application au front (6) qui coopère avec un dispositif de masque respiratoire (1, 3) de manière à ce que la position d'application du dispositif d'électrodes (9, 10, 11) est définie dans la zone frontale en fonction de la position d'application du dispositif de masque respiratoire (1, 3).

2. Dispositif selon la revendication 1, **caractérisé en ce que** l'élément d'application au front (6) est raccordé au dispositif de masque respiratoire (1, 3).

3. Dispositif selon les revendications 1 ou 2, **caractérisé en ce que** l'élément d'application au front (6) est formé dans un matériau élastomère.

4. Dispositif selon l'une quelconque des revendications 1 à 3, **caractérisé en ce que** l'élément d'application au front (6) est formé d'une pièce avec un corps de base du masque (1) du dispositif de masque respiratoire (1, 3).

5. Dispositif selon l'une quelconque des revendications 1 à 4, **caractérisé en ce qu'**un élément de rigidification est prévu qui raccorde de manière à les rigidifier l'élément d'application au front (6) et le dispositif de masque respiratoire (1, 3).

6. Dispositif selon l'une quelconque des revendications 1 à 5, **caractérisé en ce que** le dispositif d'électrodes (9, 10, 11) présente au moins deux éléments d'électrodes (9, 10).

7. Dispositif selon l'une quelconque des revendications 1 à 6, **caractérisé en ce que** le dispositif d'électrodes (9, 10, 11) présente trois éléments d'électrodes (9, 10,11).

8. Dispositif selon l'une quelconque des revendications 1 à 7, **caractérisé en ce que** les éléments d'électrodes (9, 10, 11) sont posés de manière à pouvoir céder dans une direction essentiellement verticale par rapport à la surface d'application.

9. Dispositif selon l'une quelconque des revendications 1 à 8, **caractérisé en ce que** les éléments d'électrodes (9, 10, 11) sont raccordés à un dispositif de traitement de signaux (16).

10. Dispositif selon l'une quelconque des revendications 1 à 9, **caractérisé en ce que** le dispositif de traitement de signaux (16) est intégré dans l'élément d'application au front (6).

11. Dispositif selon l'une quelconque des revendications 1 à 10, **caractérisé en ce que** le dispositif de traitement de signaux (16) est pourvu d'un dispositif de transmission de données pour la transmission sans fil des signaux traités à un dispositif de traitement de données.

12. Dispositif selon l'une quelconque des revendications 1 à 11, **caractérisé en ce que** le corps du masque (1) et l'élément d'application au front (6) sont adaptés au contour du visage du patient par un renfort présentant un dispositif de rigidification s'étendant jusqu'à l'intérieur de l'élément d'application au front (6).

13. Dispositif selon au moins une des revendications 1 à 12 pour la détection de potentiels électriques dans la zone frontale d'un patient, destiné notamment à la détermination des stades de sommeil, comportant :
un agencement de circuit de mesures pour la production de données de mesure en fonction des potentiels électriques détectés par le dispositif d'électrodes (9, 10, 11), l'agencement de circuit de mesure étant intégré dans un élément d'application au front (6), et un dispositif de transmission de signaux (16) étant prévu pour la transmission sans fil des données de mesure produites par l'agencement de circuit de mesure.

14. Dispositif selon la revendication 13, **caractérisé en ce que** l'agencement de circuit de mesure présente un dispositif pour la compression de données pour la transmission d'un jeu de données comprimé à un dispositif de transmission de signaux (16).

15. Dispositif selon au moins une des revendications 1 à 12 pour la détection de potentiels électriques dans la zone frontale d'un patient, notamment pour la détermination des stades de sommeil, comportant :
un agencement de circuit de mesure pour la production de données de mesure en fonction des potentiels électriques détectés par le dispositif d'électrodes (9, 10, 11), l'agencement de circuit de mesure étant intégré dans un élément d'application au front (6) et un dispositif d'enregistrement de données de mesure (29) étant prévu pour l'enregistrement des données de mesure produites par l'agencement de circuit de mesure.

16. Dispositif selon la revendication 15, **caractérisé en ce que** le dispositif d'enregistrement de données de mesure (29) est formé par un élément de carte de mémoire de la taille d'un timbre-poste environ qui est appliqué de manière à pouvoir être détaché.
